# EUROPEAN PATENT APPLICATION

(11) **EP 3 090 750 A1**
(43) Date of publication of application: **09.11.2016**
(21) Application number: 14876022.6
(22) Date of filing: 23.12.2014
(51) Int. Cl.: A61K 33/26, A61P 7/06

(54) **PHARMACEUTICAL COMPOSITION FOR PREVENTING OR TREATING IRON DEFICIENCY, COMPRISING IRON OXIDE NANOPARTICLES**

(30) Priority: 30.12.2013 KR 20130167673
(71) Applicant: Hanwha Chemical Corporation, Seoul 04541 (KR)
(72) Inventor: JEON, Bong Sik, Daejeon 305-315 (KR); KWON, Eun Byul, Daejeon 305-720 (KR); KIM, Eung Gyu, Daejeon 305-330 (KR); MYEONG, Wan Jae, Daejeon 305-739 (KR); PARK, Ju Young, Daejeon 305-804 (KR)
(74) Representative: Alt, Michael
(86) International application number: PCT/KR2014/012745
(87) International publication number: WO 2015/102289

(57) **Abstract**

Provided are a pharmaceutical composition including iron oxide nanoparticles for preventing or treating iron deficiency and iron deficiency anemia accompanied thereby, and a preparation method thereof.

## Description

### [Technical Field]

The present invention relates to a pharmaceutical composition for preventing or treating iron deficiency and anemia accompanied thereby, the composition including iron oxide nanoparticles, and a preparation method thereof.

### [Background Art]

Iron is an essential trace element required for almost all living organisms, and iron deficiency and excess function to inhibit or reduce cell functions in animals. In general, iron-containing materials in the body are largely divided into functional iron having metabolic and enzymatic functions and storage iron used in transport and storage. Iron deficiency is a main cause of anemia, and about 15% of the global population have iron deficiency anemia (IDA).

Iron-deficiency anemia is known as one of the most common pathologic conditions occurring in humans worldwide. Iron deficiency anemia may be generally prevented or treated by oral administration of iron-containing preparations, which is the easiest way for patients. However, there are problems that oral administration of iron preparations may cause digestive disorders, and bioavailability of the administered iron preparations is low.

Further, iron-containing preparations for parenteral administration must satisfy the requirements of easy availability of iron in hemoglobin synthesis, no topical or systemic side effects, and sufficient storage stability due to half-life. Currently available parenteral iron preparations approved for use in the U.S. include iron-dextran (e.g., InFed, Dexferrum), iron-gluconate complex (Ferrlecit), iron-sucrose (Venoferrum), etc.

Iron-dextran is a parenteral iron preparation first marketed in the U.S., and has high incidence of anaphylactic (anaphylactoid) reactions (dyspnea, asthmatic cough, chest pain, hypotension, rashes, angioedema). Iron-dextran frequently causes severe and life-threatening reactions, and also symptoms such as joint pain, back pain, hypotension, fever, muscle pain, itching, dizziness, and nausea. This high incidence of anaphylactic reactions is believed to be caused by the formation of antibodies against the dextran moiety. Even though these adverse events are not severe enough to threaten the life, further administration is precluded in many cases.

Other parenteral iron preparations (e.g., iron-sucrose and iron-gluconate) are products containing no dextran, and use of these products shows a remarkably low incidence of anaphylaxis. However, these compounds have a low iron binding capacity, and thus show high free iron concentrations in the products. For this reason, administration dose and rate are restricted according to physical properties of toxicity, high pH, and high molarity of the iron compounds, when administered. There is also a disadvantage that injectable high-molecular weight materials cause higher allergic reactions than low-molecular weight materials.

Accordingly, ferumoxytol, based on not iron-complex but iron oxide nanoparticles, was developed as an iron-containing preparation for parenteral administration, and information about its efficacy and administration is described in [Landry et al. (2005) Am J Nephrol 25, 400-410, 408], [Spinowitz et al. (2005) Kidney Intl 68, 1801-1807] and US Patent NO. 6,599,498. Ferumoxytol has a relatively large size, in which an average particle size of the iron oxide core is about 7 nm and its molecular weight is 731 kDa. Ferumoxytol has a low free iron concentration, because it has higher stability than other parenteral iron preparations such as iron-dextran, iron-sucrose, iron-gluconate, etc. A typical ferumoxytol therapy involves administration twice a week for administration of 1 g of iron, and this administration mode increases hospital costs such as tube and infusion and causes inconvenience to patients.

Iron oxide nanoparticles applicable to a variety of nano-bio fields such as contrast agents for magnetic resonance imaging (MRI), cell sorting, hyperthermia, drug delivery, biosensors, etc., may be prepared by coprecipitation, hydrothermal synthesis, thermal decomposition, etc. Among them, coprecipitation and hydrothermal synthesis are precipitation methods of directly reacting iron (I) chloride and iron (III) chloride in an aqueous solution, and these methods are used to easily prepare iron oxide nanoparticles. However, there is a disadvantage that it is difficult to control their size.

Korean Patent Publication NO. 10-2007-0102672 suggests a pyrolysis method of preparing iron oxide nanoparticles with a uniform particle size by using non-toxic metal salts as reactants, but this method has a disadvantage that complicated conditions are required for the preparation of iron oxide nanoparticles with a particle size of 4 nm or less, and thus commercialization is difficult. Further, Korean Patent Publication NO. 10-2012-0013519 discloses a method of preparing iron oxide nanoparticles with a size of 4 nm or less, but its use is limited to T1 contrast agents.

Further, iron oxide nanoparticles, for example, triiron tetraoxide (Fe₃O₄, magnetite) nanoparticles are easily precipitated in the body, and therefore, surface treatment has been performed using polymers such as dextran or chitosan, before use. However, the use of dextran increases incidence of anaphylactic reaction which is one of immediate hypersensitivity reactions, leading to life-threatening situations. Further, use of non-dextran materials such as chitosan has a disadvantage of low iron delivery to the body due to their low iron binding capacity.

To solve these disadvantages of the prior art, accordingly, there is an urgent need for a pharmaceutical composition for preventing or treating iron deficiency or anemia accompanied thereby, including iron oxide nanoparticles, in which the composition is non-toxic to human body, and has low anaphylactic reactions and high efficiency of iron delivery.

### [Disclosure]

### [Technical Problem]

An object of the present invention is to provide a pharmaceutical composition for preventing or treating iron deficiency, including iron oxide nanoparticles as an active ingredient, in which the composition has no toxicity caused by a high concentration of free iron upon parental administration, has low incidence of anaphylactic reactions, high iron bioavailability, and long-term storage stability at room temperature.

Another object of the present invention is to provide a method of preparing the pharmaceutical composition, the method including the steps of reacting iron oxide nanoparticles with phosphate or phosphate-polyethylene glycol and then dispersing the resulting iron oxide nanoparticles in water to obtain a nanoparticle aqueous solution; freeze-drying the nanoparticle aqueous solution to obtain dry nanoparticles; and dispersing the dry nanoparticles in a saline solution, followed by concentration.

### [Technical Solution]

In an aspect to achieve the above objects, the present invention provides a pharmaceutical composition for preventing or treating iron deficiency or iron deficiency anemia accompanied thereby, the composition including iron oxide nanoparticles.

In the present invention, the iron oxide nanoparticles may be those having iron oxide as a core and being surface-modified with phosphate-polyethylene glycol (PO-PEG), in which the iron oxide may be one or more selected from the group consisting of iron (II) oxide (FeO), iron (III) oxide (Fe₂O₃) and triiron tetraoxide (Fe₃O₄), and the iron oxide nanoparticles may be paramagnetic or pseudo-paramagnetic.

Another aspect of the present invention is to provide a method of preparing the pharmaceutical composition, the method including the steps of reacting iron oxide nanoparticles with phosphate or phosphate-polyethylene glycol (PO-PEG) and then dispersing the resulting iron oxide nanoparticles in water to obtain a nanoparticle aqueous solution; freeze-drying the nanoparticle aqueous solution to obtain dry nanoparticles; and dispersing the dry nanoparticles in a saline solution, followed by concentration.

According to the present invention, when the surface of the iron oxide particles is modified with phosphate or a complex of phosphate-polyethylene glycol (PO-PEG), efficiency of iron delivery to the body may be increased, biocompatibility may be improved to show no toxicity, and stability may be increased to allow long-term storage at room temperature.

### [Advantageous Effect]

A pharmaceutical composition for preventing or treating iron deficiency including iron oxide nanoparticles as an active ingredient according to the present invention may overcome digestive disorders and low bioavailability upon oral administration, and also minimize a toxicity problem of free iron due to dissociation of iron preparations, in particular, anaphylactic reactions upon parenteral administration. Further, the present invention improves stability of the iron oxide nanoparticles to allow long-term storage at room temperature, and also provides iron preparations applicable to bolus injection, thereby greatly improving patient convenience.

### [Brief Description of Drawings]

FIG. 1 shows a transmission electron microscopic image of iron oxide nanoparticles with a size of 3 nm, which were prepared in Example 1-1 of the present invention;
FIG. 2 shows changes in iron (Fe-59) distributions in organs over time, after administration of mice with KEG3 which is a pharmaceutical composition prepared in Example 1 of the present invention;
FIG. 3 shows changes in iron (Fe-59) distributions in plasma and blood cells, after administration of mice with KEG3 which is the pharmaceutical composition prepared in Example 1 of the present invention;
FIG. 4 shows a comparison of changes in blood hemoglobin (Hb) levels over time between an excipient-treated group and a KEG3-treated group prepared by administration of normal mice with KEG3 which is the pharmaceutical composition of Example 1 of the present invention;
FIG. 5 shows a comparison of changes in the mean cell volume (MCV) of red blood cells over time between an excipient-treated group and a KEG3-treated group prepared by administration of normal mice with KEG3 which is the pharmaceutical composition of Example 1 of the present invention; and
FIG. 6 is a graph showing a comparison of free iron concentrations between commercial anemia drugs and KEG3 which is the pharmaceutical composition of Example 1 of the present invention.

### [Best Mode]

Hereinafter, the present invention will be described in more detail.

The present invention relates to a pharmaceutical composition for preventing or treating iron deficiency or iron deficiency anemia accompanied thereby, the composition including iron oxide nanoparticles.

As used herein, the term 'iron oxide' refers to a material formed by binding of iron with oxygen. Examples of the iron oxide may include iron oxide such as iron (II) oxide (FeO), iron (III) oxide (Fe₂O₃), and triiron tetraoxide (Fe₃O₄, magnetite) and iron metal oxide, but are not limited thereto.

In an embodiment of the present invention, the iron oxide nanoparticles may be those surface-treated with a hydrophilic material, and for example, the surface of the iron oxide particles is modified with phosphate or phosphate-polyethylene glycol (PO-PEG) to increase efficiency of iron delivery to the body. Particularly, such coating materials have excellent biocompatibility to show no toxicity, and increase stability to allow long-term storage at room temperature.

With regard to the nanoparticles of the present invention, the iron oxide core particle may have an average particle size of 1 nm or more to 4 nm or less, and preferably, 2 nm or more to 4 nm or less. To use the iron oxide nanoparticles of the present invention as the active ingredient of the pharmaceutical composition for preventing or treating iron deficiency or iron deficiency anemia, it is necessary to prepare the iron oxide nanoparticles in a small and uniform size.

In view of excretion rate and bioavailability of the iron oxide nanoparticles, the particle size of the iron oxide core of the nanoparticles may be preferably 1 nm or more. If the particle size exceeds 4 nm to increase the volume of the iron oxide nanoparticles, the time taken for particle decomposition and bioavailability is increased, and translocation of iron oxide nanoparticles to other organs than blood vessels is increased to reduce availability in the blood.

In a specific embodiment, the iron oxide nanoparticles of the present invention may be paramagnetic or pseudo-paramagnetic, and for example, the iron oxide nanoparticles may be paramagnetic or pseudo-paramagnetic at a temperature of 20 K or higher.

Specifically, the iron oxide nanoparticles according to the present invention may be a) paramagnetic or pseudo-paramagnetic at a temperature of 20 K or higher, b) may have iron oxide core particles with an average particle size of 1 nm to 4 nm, c) may be surface-treated with a hydrophilic material, and d) may have no agglomeration between particles. Preferably, the hydrophilic material may be phosphate or phosphate-polyethylene glycol (PO-PEG).

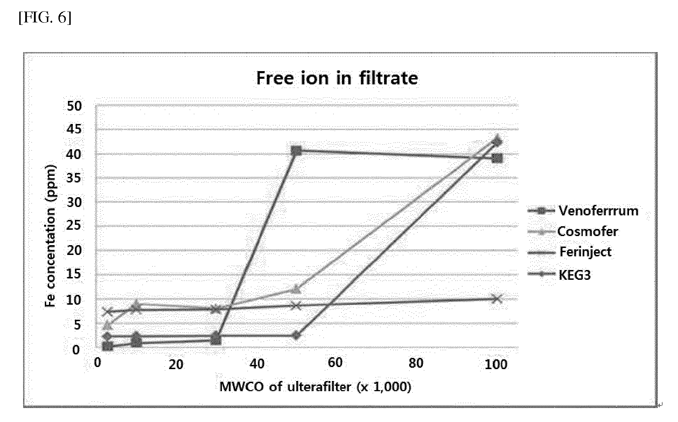

The iron oxide nanoparticles according to the present invention are appropriate for drugs which are used to treat and prevent iron deficiency and iron deficiency anemia accompanied thereby. In particular, free iron concentrations in the iron oxide nanoparticles according to the present invention were measured. As a result, it was found that the free iron concentrations were very low, compared to those in the existing iron preparations (FIG. 6). Therefore, because of the low free iron concentration, the composition of the present invention has little toxicity caused thereby, indicating that the iron oxide nanoparticles may be safe enough to be administered to the body.

The iron deficiency anemia, for example, iron deficiency anemia in pregnant women, latent iron deficiency anemia in children and adolescents, iron deficiency anemia due to gastrointestinal disorders, iron deficiency anemia due to blood loss, e.g., bleeding of gastrointestinal tract (caused by, for example, ulcers, cancers, hemorrhoids, inflammatory diseases, intake of acetylsalicylic acid), iron deficiency anemia caused by menstruation, iron deficiency anemia caused by injury, iron deficiency anemia due to spurue, and iron deficiency anemia due to iron deficient diet in children and adolescents who eat only what they like may be prevented or treated. Further, the pharmaceutical composition according to the present invention may be used to prevent or treat diseases caused by iron deficiency, for example, impaired immunity, cerebral dysfunction, and restless legs syndrome, as well as iron deficiency and iron deficiency anemia.

In pathological cases, a reduced serum iron level leads to a reduced hemoglobin level, reduced red blood cell production and thus to anemia. External symptoms of anemia include fatigue, pallor, lack of concentration, etc. Clinical symptoms of anemia include low serum iron levels (hypoferremia), low hemoglobin levels, low hematocrit levels, a reduced number of red blood cells, reduced reticulocytes, and elevated levels of soluble transferrin receptors.

As used herein, the term "prevention" means inhibition of occurrence of a diseases or disorder in an animal which has not been diagnosed with the disease or disorder, but is vulnerable to the disease or disorder. Further, as used herein, the term "treatment" means inhibition of development of a disease or disorder, alleviation of the disease or disorder, or elimination of the disease or disorder.

The present invention relates to a medicinal composition including the iron oxide nanoparticles according to the present invention, any one or more additional pharmaceutically effective compounds, and any one or more pharmaceutically acceptable carriers and/or an auxiliary material, and/or a solvent.

Further, the pharmaceutical composition of the present invention may be administered to children, adolescents, and adults via any route of oral and parenteral administration, and preferably, administered via a parenteral route.

As used herein, the term "administration" means introduction of the pharmaceutical composition of the present invention into a subject in need of disease treatment by a certain suitable method. The composition of the present invention may be administered via various routes including oral or parenteral routes, as long as it is able to reach a desired tissue. With respect to the objects of the present invention, the specific therapeutically effective dose level for any particular patient may vary depending on various factors well known in the medical art, including the kind and degree of the response to be achieved, concrete compositions according to whether other agents are used therewith or not, the patient's age, body weight, health conditions, gender, and diet, the time and route of administration, the secretion rate of the composition, the time period of therapy, other drugs used in combination or coincidentally with the specific composition, and like factors well known in the medical arts.

More specifically, the composition of the present invention may be given by the parenteral administration of intravenous or intramuscular bolus injection. Further, an injectable formulation for parenteral administration may include an isotonic aqueous solution or a suspension, and may be formulated according to the known method in the art by using an appropriate dispersing agent or wetting agent and a suspending agent. For example, respective ingredients may be formulated into an injectable formulation by dissolving them in a saline solution or a buffer. Further, formulations for oral administration may include, but are not limited to, powders, granules, tablets, pills, emulsions, syrups, capsules, etc.

As used herein, the term "pharmaceutically acceptable carrier" refers to a carrier or a diluent that does not cause significant irritation to an organism and does not abrogate the biological activity and properties of the administered compound. Examples of the pharmaceutically acceptable carrier may include carriers for oral administration such as lactose, starch, cellulose derivatives, magnesium stearate, stearic acid, etc., and carriers for parenteral administration such as water, suitable oil, a saline solution, aqueous glucose and glycol. These pharmaceutically acceptable carriers may include, for example, saline, sterilized water, Ringer's solution, buffered saline, a dextrose solution, a maltodextrin solution, glycerol, ethanol, or a mixture of at least one among these ingredients. Optionally, other typical additives such as a stabilizer, a preservative, an antioxidant, a buffer solution, a bacteriostatic agent, etc. may be added as an excipient.

Another aspect of the present invention provides a method of preparing the pharmaceutical composition, the method including the steps of reacting iron oxide nanoparticles with phosphate or phosphate-polyethylene glycol (PO-PEG) and then dispersing the resulting iron oxide nanoparticles in water to obtain an iron oxide nanoparticle aqueous solution; freeze-drying the iron oxide nanoparticle aqueous solution to obtain dry nanoparticles; and dispersing the dry nanoparticles in a saline solution, followed by concentration. In the dispersing and concentration steps, a pharmaceutically acceptable excipient or additive may be added.

### [Mode for Invention]

Hereinafter, the present invention will be described in detail with reference to Examples. However, the following Examples are for illustrative purposes only, and the present invention is not intended to be limited by the following Examples.

### Example 1. Preparation of Iron Oxide Nanoparticle-Containing Pharmaceutical Composition (KEG3)

### 1-1: Preparation of Iron Oxide Nanoparticles Having Particle Size of 3 nm

1.8 g (2 mmol) of iron oleate, 0.57 g (2 mmol) of oleic acid, and 1.61 g (6 mmol) of oleyl alcohol were mixed with 10 g of diphenyl ether. Then, the mixture was added to a round-bottom flask, and degassed under vacuum at 80°C for 1 hour. Argon was applied to the flask to maintain an inert atmosphere, and then reaction was allowed while the temperature was raised to 250°C at a rate of 10°C/min. As the reaction proceeded, the color of the reactants was found to change to black. After the temperature reached to 250°C, the reaction was allowed for 30 minutes, followed by rapid cooling. The product was washed with excess acetone. Precipitate obtained after washing was dispersed in an organic solvent such as chloroform or hexane.

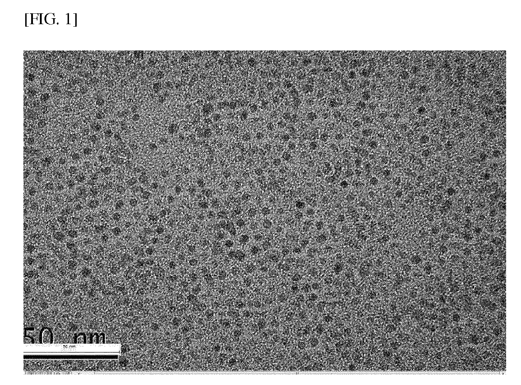

FIG. 1 is a photograph showing the result of transmission electron microscopy of the iron oxide nanoparticles of Example 1-1. The iron oxide core particle of the iron preparation had an average particle size of 3.4, and the standard deviation of the particle size was 0.38 nm.

### 1-2: Surface-treatment of Iron Oxide Nanoparticle with Phosphate-Polyethylene Glycol (Phosphate-PEG, PO-PEG)

0.15 g of phosphoryl chloride (POCl₃) and 6 g of polyethylene glycol methyl ether were added to 7 ml of Tetrahydrofuran (THF) solution, followed by agitation for 4 hours. THF was removed to obtain phosphate-polyethylene glycol (PO-PEG).

10 mg of the iron oxide nanoparticles having a particle size of 3 nm prepared in Example 1-1 were mixed with 100 mg of the prepared phosphate-polyethylene glycol (PO-PEG) in ethanol, and then sealed. Agitation was performed at 70°C for 4 hours for ligand exchanging. The resulting iron oxide nanoparticles were washed with N-hexane three times, and ethanol was evaporated, followed by addition of water. The resulting iron oxide nanoparticles were dispersed in water to obtain an aqueous solution of surface-treated iron oxide nanoparticles having a hydrodynamic diameter of 11.7 nm.

### 1-3: Preparation of Iron Oxide Nanoparticle-Containing Pharmaceutical Composition (KEG3)

100 ml of 0.9% normal saline was added to dry nanoparticles which were obtained by freeze-drying the aqueous solution of the surface-treated iron oxide nanoparticles obtained in Example 1-2, and thus the nanoparticles were dispersed therein. The dispersion was passed through a syringe filter having a pore size of 0.2 µm. The filtrate was divided and put in Amicon® ultra centrifugal filter-ultracel®-50K having a volume of 15 ml, and concentration was performed by removing the filtrate with centrifugation at 3,000 rpm for 60 minutes. 0.9% normal saline was added to each of the concentrated filters for dilution. Concentration by centrifugation was repeated several times to obtain a solution, in which iron oxide nanoparticles were concentrated in 0.9% normal saline. This solution was designated as KEG3.

### Example 2. Preparation of Fe-59-labeled KEG3

A hydrophilized nanoparticle solution was prepared in the same manner as in Example 1 by using iron oxide nanoparticles which were prepared in the same manner as in Example 1-1, except that ⁵⁹FeCl₃-added Fe-oleate complex was used. Synthesis of iron oxide nanoparticles of about 3 nm was confirmed by transmission electron microscope (TEM). The measurement result after hydrophilization showed that a hydrodynamic diameter of the particle was 14.1 nm.

Radiation intensity of the Fe-59-labeled iron oxide nanoparticles having a particle size of about 3 nm in the KEG3 pharmaceutical composition prepared in Example 1-3 was measured, and then a normal saline solution (0.9 % NaCl aqueous solution) was added to prepare a solution with an iron (Fe) concentration of 1409 µg Fe/mL.

### Comparative Example 1. Synthesis of Iron Oxide Nanoparticle Having Particle Size of 12 nm

Iron oxide nanoparticles having a particle size of 12 nm were prepared in the same manner as described in Ultra-large-scale syntheses of monodisperse nanocrystals, J. Park et al. Nature Materials 3, 891 - 895 (2004). 1.8 g of iron oleate and 0.28 g of oleic acid were mixed with 10 g of 1-octadecene, followed by raising temperature to 318°C at a rate of 3.3°C/min and growing particles at 318°C for 30 minutes.

### Example 3. Iron Oxide Distribution by Fe-59-labeled KEG3 in Mouse

0.1 ml of Fe-59-labeled KEG3 solution prepared in Example 3 was injected via the tail vein of 6-week-old ICR mice. The blood, muscle, bone, fat, heart, liver, lung, kidney, intestine, pancreas, spleen, and stomach (n=3 ~ 4) were collected 5, 10, and 30 minutes, 1 and 6 hours, 1, 3, 10, 30, 91 and 182 days post-injection. The contents of nanoparticles in the blood and various organs were calculated by measuring ⁵⁹Fe radioactivity using a gamma counter. To evaluate exposure of the iron oxide nanoparticles to the respective organs by intravenous administration, the radioactivity measured in the organs was quantified as %ID(injection dose)/g and %ID/organ (%ID: percentage of remaining dose to injected dose (⁵⁹Fe), %ID/g: %ID per unit weight, %ID/organ: %ID per organ). The respective organs were removed and weighed. Since it is difficult to measure the entire weights of the blood, muscle, fat, and bone, their proportions to the total body weight were used to calculate their weights (blood: 7 %, muscle: 40 %, fat: 7 %, bone: 10 %). Further, in order to measure ⁵⁹Fe distribution in the plasma and red blood cell in the blood, the blood was collected over time, and then plasma and red blood cells were separated therefrom. Radioactivities of the plasma and red blood cells were measured.

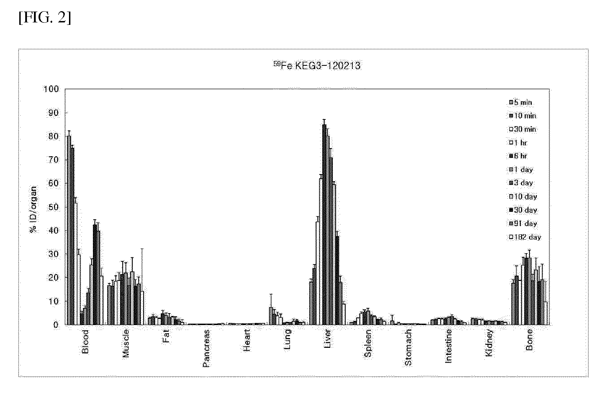

As shown in FIG. 2, uptake of iron oxide nanoparticles was mainly found in the blood, liver, and spleen. In the liver, %ID in the organ increased until 6 hrs, and then continuously decreased after 6 hours. The liver showed 3.73 %ID/g and 8.88 %ID/organ at 182 days. The exposure to the spleen showed 12.4 %ID/g and 1.39 %ID/organ at 182 days, as similar to the liver. To calculate the percentage of the remaining iron oxide nanoparticles in the body, a value obtained by subtracting the content of ⁵⁹Fe measured in the red blood cells from the content of ⁵⁹Fe measured in the whole body at the corresponding time was assessed. In this regard, there is a concern that calculation of the radiation intensity in the muscle, fat, or bone may be duplicated with calculation of the radiation intensity in the blood, and it is also difficult to measure the entire weights thereof. Therefore, the muscle, fat, and bone were excluded from the calculation of the radiation intensity of the whole body. After administration at a dose of 5.2 mg Fe/kg, about 33% and 64% of the administration dose were excreted 91 and 182 days post-administration, respectively.

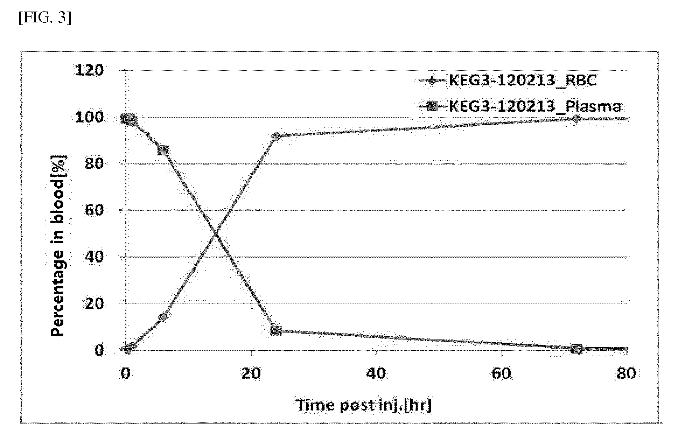

As shown in FIG. 3 (graph of ⁵⁹Fe concentration comparison of plasma and red blood cell), ⁵⁹Fe in the plasma gradually decreased whereas ⁵⁹Fe in the red blood cells gradually increased over time. After 3 days, most ⁵⁹Fe present in the blood were in the red blood cells.

### Example 4. Pharmacokinetic Studies of KEG3 in rats

The KEG3 solution prepared in Example 1 was intravenously administered to 45 specific pathogen-free (SPF) Sprague-Dawley rats [Crl:CD(SD)] at a single dose of 2.6, 5.2, or 10.4 mg Fe/kg, and blood was collected at a predetermined time. The blood was centrifuged to obtain only the plasma, and T2 relaxation time of the plasma was measured in a 4.7T magnetic resonance imaging (MRI) scanner to analyze the KEG3 concentration in the blood.

During the experiment, no animal deaths and abnormalities were observed, and pharmacokinetic parameters of KEG3 in rats were given in the following Table 1. In the experimental groups prepared by administering male rats with KEG3 at a dose of 2.6, 5.2 or 10.4 mg Fe/kg, the systemic exposure (as determined by AUC_{inf}) increased at a ratio of about 1:3:7 in a dose-dependent manner, as the administration dose increased at a ratio of 1:2:4. In a concentration-time curve after administration of the test material, terminal half-life (t_{1/2}) was 2.2-4.2 hr in all administration groups. 12-24 hr after administration of the test material, the concentrations were below the limit of quantitation (3 µg Fe/mL).

**[Table 1]**

| Group | Dose | Gender | Kₑ₁ | T_{1/2} | AUCₗₐₛₜ | AUC_{inf} | Tₘₐₓ | Cₘₐₓ | CL |
|---|---|---|---|---|---|---|---|---|---|
| | (mg Fe/kg) | | (1/h) | (h) | (µg Fe-h/mL) | (µg Fe-h/mL) | (h) | (µg Fe/mL) | (mL/h/kg) |
| T1 | 2.6 | Male | 0.31 | 22 | 287.6 | 296.3 | 0.033 | 66.6 | 8.8 |
| T2 | 5.2 | Male | 0.17 | 4.2 | 735.2 | 829.4 | 0.083 | 140.9 | 6.3 |
| T3 | 10.4 | Male | 0.18 | 39 | 2163.9 | 2207.4 | 0.083 | 276.2 | 4.7 |

| | | | | | | | | | |
|---|---|---|---|---|---|---|---|---|---|
| Pharmacokinetic parameters: apparent terminal elimination rate constant (Kₑₗ), terminal half life (t_{1/2}), maximum observed peak serum concentration (Cₘₐₓ), time at which Cₘₐₓ is observed (Tₘₐₓ), total clearance (CL), area under the serum concentration time curve (AUC) from the start of dosing to the time of last sampling (AUCₗₐₛₜ) the area under the plasma concentration-time curve from 0 h to infinity (AUC_{inf}) with an extrapolation to time infinity. | | | | | | | | | |

### Example 5. Influence of KEG3 on blood components in normal mice

To analyze the influence of KEG3 of Example 1 in normal mice, 42 9-week-old C57BL/6 mice were intravenously administered with an excipient or KEG3 prepared in Example 1 at a dose of 5.2 mg Fe/kg. On the day of administration and 1, 3, 7 and 14 days after administration, blood was collected, and changes in the blood components were analyzed to examine influence of the iron oxide nanoparticles.

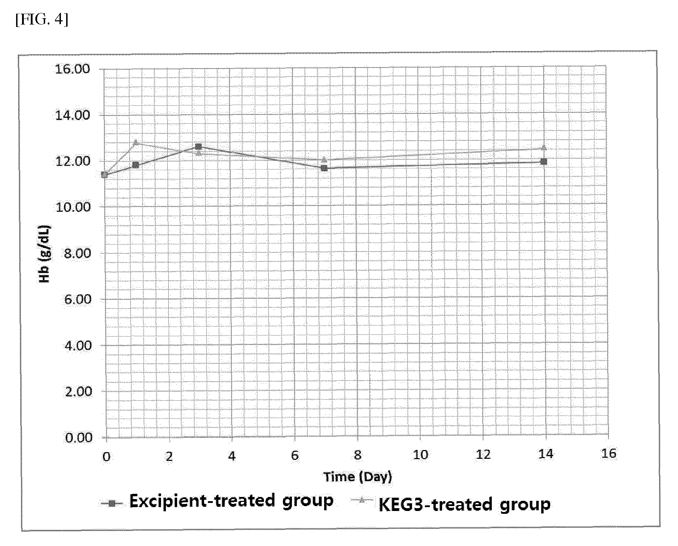

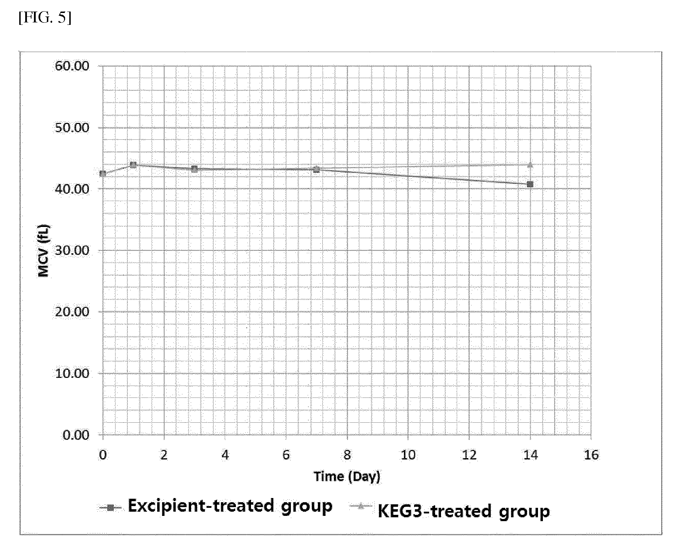

During the experimental period, no abnormalities were found in all experimental animals. As shown in FIG. 4, there was no significant change in the blood hemoglobin (Hb) level of the KEG3-treated group, compared to the excipient-treated group. As shown in FIG. 5, as compared to the excipient-treated group, the KEG3-treated group showed a statistically significant increase (p<0.05) in the mean cell volume (MCV) of red blood cells, but satisfying the MCV of normal blood, at 14 days after administration of the test material. Therefore, it was confirmed that there was no great change in the blood components of normal mice by KEG3 administration.

### Example 6. Test of Free Iron Concentration of KEG3

To example the concentration of free iron which generates toxicity during parenteral administration of KEG3 prepared in Example 1, free iron concentrations of commercial iron preparations and KEG3 of Example 1 were examined by ultrafiltration.

Three commercial anemia drugs (Venoferrum, Cosmofer, Ferinject) and KEG3 prepared in Example 1 were diluted at a concentration of 2,000 µgFe/mL, respectively. Then, each of them was centrifuged at 3,000 rpm for 30 minutes using centrifugal filters with molecular weight cutoffs (MWCO) of 10,000, 30,000, 50,000 and 100,000 to collect only the filtrates.

50 µL of 0.5 M citrate buffer containing 10 mM ascorbic acid was added to 500 µL of the solution, and allowed to react for 30 minutes. 10 µL of 50 mM BPS (Bathophenantroline disulfonate) was added thereto, and allowed to react for 20 minutes or longer. Each 100 µL of the solution was added to a 96-well microplate, and absorbance at 535 nm was measured using a microplate reader to determine free iron concentrations.

As shown in FIG. 6, the free iron concentrations of KEG3 prepared in Example 1 showed uniform levels in the filters of 50,000 or less, irrespective of the molecular weight cutoff, and the free iron concentrations were also lower than those of the commercial anemia drugs, indicating that KEG3 has a low toxicity.

### Example 7. Acute Toxicity Test of Single Intravenous Administration in Rat

5 specific pathogen-free (SPF) Sprague-Dawley rats [Crl:CD(SD)] were used to examine acute toxicity of single intravenous administration of KEG3. As an administration route of the test material, intravenous administration which is a clinical intended route in humans was performed. In the test, an administration dose was a maximum dose of 840 mg/kg, based on a maximum single injection volume of 10 mL/kg. By applying a dose progression factor of 3.2, the next doses were determined as administration doses (1.75, 5.5, 17.5, 55, 175, 550 and 840 mg/kg) recommended in OECD Guideline No.425. The next dose was determined according to the procedure of the limit dose test recommended by AOT425statPgm. If an animal survived after single administration of a limit dose of 840 mg/kg, a next animal was administered with the same dose. If 3 or more out of 5 mice died, the test was changed to the main test. Constitution of single dose toxicity test groups and administration doses are given in the following Table 2.

**[Table 2]**

| Animal No. | Gender | Number of animals | Administration amount (mL/kg) | Administration dose (mg/kg/day) |
|---|---|---|---|---|
| 1* | Female | 1 | 10 | 840 |
| 2 | Female | 1 | 10 | 840 |
| 3 | Female | 1 | 10 | 840 |
| 4 | Female | 1 | 10 | 840 |
| 5 | Female | 1 | 10 | 840 |

| | | | | |
|---|---|---|---|---|
| *Animal administered with a starting dose | | | | |

During the experimental period, no animal death was observed, and changes in skin color and colored urine related to administration of test materials were observed. Inhibitions of weight loss and gain were observed in all animals. No gross findings related to administration of test materials were observed. A lethal dose 50 (LD50) of the test material by intravenous administration of female rats with the iron oxide nanoparticles was found to exceed 840 mg/kg (300 mg Fe/kg).

## Claims

1. A pharmaceutical composition for preventing or treating iron deficiency or iron deficiency anemia, comprising iron oxide nanoparticles.

2. The pharmaceutical composition of claim 1, wherein the iron oxide nanoparticle comprises a core particle of iron oxide and a surface modified with phosphate-polyethylene glycol.

3. The pharmaceutical composition of claim 1, wherein the core particle of the iron oxide nanoparticle has a size of 1 nm to 4 nm.

4. The pharmaceutical composition of claim 1, wherein the iron oxide is one or more selected from the group consisting of iron (II) oxide (FeO), iron (III) oxide (Fe₂O₃), and triiron tetraoxide (Fe₃O₄).

5. The pharmaceutical composition of claim 1, wherein the iron oxide nanoparticles are paramagnetic or pseudo-paramagnetic.

6. The pharmaceutical composition of claim 1, wherein the iron oxide nanoparticles are paramagnetic or pseudo-paramagnetic at a temperature of 20 K or higher,
comprises iron oxide core particles with a particle size of 1 nm to 4 nm and c) surface treated with a hydrophilic material, and
have no agglomeration between particles.

7. The pharmaceutical composition of claim 6, wherein the hydrophilic material is phosphate-polyethylene glycol.

8. The pharmaceutical composition of claim 1, wherein the iron deficiency anemia is one or more selected from the group consisting of iron deficiency anemia in pregnant women, latent iron deficiency anemia in children and adolescents, iron deficiency anemia due to gastrointestinal disorders, iron deficiency anemia due to blood loss, iron deficiency anemia caused by menstruation, iron deficiency anemia caused by injury, iron deficiency anemia due to spurue, iron deficiency anemia of cancer patients, iron deficiency anemia due to chemotherapy, iron deficiency anemia caused by inflammation (AI), iron deficiency anemia caused by chronic heart failure (CHF), iron deficiency anemia caused by rheumatoid arthritis (RA), iron deficiency anemia caused by systemic lupus erythematosus (SLE), and iron deficiency anemia caused by inflammatory bowel disease (IBD).

9. The pharmaceutical composition of claim 1, wherein the pharmaceutical composition is administered by intravenous or intramuscular bolus injection.

10. The pharmaceutical composition of claim 1, further comprising a pharmaceutically acceptable excipient or carrier.

11. A method of preparing a pharmaceutical composition comprising iron oxide nanoparticles, the method comprising the steps of:
reacting iron oxide nanoparticles with phosphate-polyethylene glycol and dispersing the resulting iron oxide nanoparticles in water to obtain a nanoparticle aqueous solution;
freeze-drying the nanoparticle aqueous solution to obtain dry nanoparticles; and
dispersing the dry nanoparticles in a saline solution and performing concentration.
